# EUROPEAN PATENT APPLICATION

(11) **EP 3 912 633 A1**
(43) Date of publication of application: **24.11.2021**
(21) Application number: 21173842.2
(22) Date of filing: 14.05.2021
(51) Int. Cl.: A61K 36/19, A61P 31/14, A61K 31/724, A61K 47/69

(54) **BIOACTIVE FORMULATION AND METHOD FOR PREPARATION THEREOF**

(30) Priority: 16.05.2020 IN 202011020695
(71) Applicant: Ambe Phytoextracts Pvt. Ltd., 246276 Village - Jamriya (IN)
(72) Inventor: Singh, Harshpal, 246276 Village - Jamriya (IN); Singh, Kul Bhaskar, 246276 Village - Jamriya (IN); Singh, Shashi Chandrama, 246276 Village - Jamriya (IN); Choudhary, Muskan, 246276 Village - Jamriya (IN)
(74) Representative: Petraz, Gilberto Luigi

(57) **Abstract**

A bioactive formulation comprising ethanolic concentrates of Andrographolide extract in range of 90% by wt - 99% by wt and aqueous 2 hydroxypropyl beta cyclodextrin in the range of 2% by wt - 10% by wt. A method for preparation of the bioactive formulation comprising the following steps: mixing Andrographolide extract and ethanol to prepare ethanolic concentrates of Andrographolide (solvent A), mixing hydroxypropyl beta cyclodextrin and ethanol to obtain an ethanolic concentrate 2 hydroxypropyl beta cyclodextrin (Solvent B), mixing the solvent A and solvent B to obtain a complex concentrate solution, recovering ethanol solvent from the concentrate solution through distillation to obtain a thick paste of the concentrate, drying the paste at a temperature in the range of 55°C - 65°C and pressure in the range of -650 to 700mm Hg to obtain a dried complex concentrate followed by converting into an amorphous powder to obtain a bioactive formulation.

## Description

### FIELD OF THE INVENTION

The present invention relates to a field of healthcare formulations. More specifically, the present invention relates to a bioactive formulation and method for preparing the formulation by synergistic combination of Andrographolide (AG) extract with Hydroxy propyl beta cyclodextrin (HPBCD) to improve the bioavailability and solubility and to boost the immune system of the person thereby proving potency of the formulation having antiviral activities against SARS-CoV-2 infection and COVID-19 disease.

### BACKGROUND OF THE INVENTION

Corona virus belongs to large family of viruses that leads to development of illness which includes common cold to more severe diseases such as Middle East Respiratory Syndrome (MERS-CoV) and severe acute respiratory syndrome (SARS-CoV). The virus specifically attacks the respiratory tract which if left untreated weakens the immune system.. The virus particles is transmitted from human to human by a means of contaminated airborne particles.

The immune system comprises of a group of complex cells, procedures, and synthetic substances that continuously protects the body against pathogens including viruses, toxic agents, and harmful bacteria that invade in the body and try to replicate using the host's machinery. People with prior ailments like diabetes, hypertension, cardio vascular infection, and respiratory issues are more prone to infection from COVID- 19. Experts has advised that supplementing the body with specific nutrients, minerals, herbs, and different substances may boost the immune system and conceivably provide resistance against foreign substances that invade our body.

US8911724B2 discloses about methods and compositions for increasing CD4+ cell count in mammals, preferably by at least 25%, while undergoing treatment for various conditions whose treatment can have detrimental effects on immune system function. Such conditions include, but are not limited to, HIV infection, cancer, and hepatitis. The compositions include alpha lipoic acid; acetyl L-carnitine; N-acetylcysteine; and optionally: zinc, selenium, vitamin C, bioflavinoid complex, vitamin E, co-enzyme Q10, glutathione, beta-carotene, vitamin A, vitamin B1, vitamin B2, vitamin B6, niacinamide, calcium pantothenate, folic acid, vitamin B12, copper, manganese, chromium, and molybdenum.

Many formulations are available in the market that are used to enhance the immune system of the person but still no such formulation has proven to be effective against SARS-CoV 2 Viral infection as well as clinical management of symptoms of COVID-19 disease.

Therefore, there is a need to provide a formulation that possess anti-viral as well as immunomodulatory properties to boost the immune system as well as provide resistance against infection from SARS-CoV 2 Virus.

### OBJECTS OF THE INVENTION

The principal object of the present invention is to overcome the disadvantages of the prior art.

An object of the present invention is to provide a bioactive formulation and method for preparation by synergistic combination of Andrographolide (AG) extract with Hydroxy propyl beta cyclodextrin (HPBCD) to enhance the immunity of the subject and capable of being utilized for prophylaxis as well as clinical management of SARS-CoV 2 infection and COVID-19 disease.

Another object of the present invention is to provide a bioactive formulation - having improved Solubility & Bioavailability.

Another object of the present invention is to provide a bioactive formulation derived from a natural extract and possess antiviral activities against SAR-CoV-2 Virus as well as possess immunomodulatory properties.

Another object of the present invention is to provide a bioactive formulation - combination of Andrographolide (AG) extract with Hydroxy propyl beta cyclodextrin (HPBCD), to be proven to be nontoxic and safe for human consumption via oral or nasal route.

Yet another object of the present invention is to provide a formulation that may be administered orally as well as via pulmonary delivery.

The foregoing and other objects, features, and advantages of the present invention will become readily apparent upon further review of the following detailed description of the preferred embodiment as illustrated in the accompanying drawings.

### SUMMARY OF THE INVENTION

The present invention is directed towards a formulation and method for preparation of a herbal anti-viral and immunomodulating formulation by combining Andrographolide (AG) extract and Hydroxy propyl beta cyclodextrin (HPBCD) for managing the clinical symptoms of various health ailments such as upper respiratory tract infections as well as inhibiting SARS-CoV 2 viral infection and clinical symptoms of COVID-19 disease.

According to an embodiment of the present invention, a bioactive formulation comprises of : i) ethanolic concentrates of Andrographolide extract in the range of 90% by wt- 99% by wt, and ii) aqueous 2 hydroxypropyl beta cyclodextrin (HPBCD) in the range of 2% by wt- 10% by wt. The Andrographolide (AND) is extracted from *Andrographis paniculata.*

According to an embodiment of the present invention, a method for preparation of a bioactive formulation comprises of the following steps: i) adding the Andrographolide extract and ethanol in a first beaker followed by mixing to obtain ethanolic concentrates of Andrographolide (solvent A), ii) adding the hydroxypropyl Beta cyclodextrin (HPBCD) and ethanol in a second beaker followed by mixing to obtain an ethanolic concentrates of 2 hydroxypropyl beta cyclodextrin (Solvent B), iii) mixing solvent A and solvent B for 1-3 hour at room temperature to obtain a complex concentrate solution i.e. Andrographolide and 2 hydroxypropyl beta cyclodextrin complex, iv) recovering ethanol solvent from the concentrate solution through distillation to obtain a thick paste of the complex concentrate, v) drying the thick paste in a vacuum tray drier at a temperature in the range of 55°C-65 °C and pressure in the range of -650to 700mmHg to obtain a dried complex concentrate, and vi) converting the dried complex concentrate into an amorphous powder to obtain a final bioactive formulation.

While the invention has been described and shown with particular reference to the preferred embodiment, it will be apparent that variations might be possible that would fall within the scope of the present invention.

### BRIEF DESCRIPTION OF THE DRAWINGS

These and other features, aspects, and advantages of the present invention will become better understood with regard to the following description, appended claims, and accompanying drawings where:
**Figure 1** illustrates a flow chart representation of method for preparation of bioactive formulation;
**Figure 2 a****,** **b****,** **c** illustrates a graphical representation of ¹H NMR spectra of Andrographolide, 2-HP-β-CD, bioactive formulation;
**Figure 3** illustrates a graphical representation of 2D NMR (ROSEY) of the bioactive formulation;
**Figure 4** illustrates a pictorial representation of in silico docking analysis of the bioactive formulation;
**Figure 5** illustrates a graphical representation of differential scanning electroscopy;
**Figure 6** illustrates a graphical representation of dissolution rate of the bioactive formulation;
**Figure 7** illustrates a graphical representation of PXRD and SEM analysis of Andrographolide, 2-HP-β-CD, physical mixture of Andrographolide with 2-HP-β-CD (1:1 mM), and bioactive formulation; and
**Figure 8** illustrates a graphical representation of pharmacokinetic analysis of the bioactive formulation.

### DETAILED DESCRIPTION OF THE INVENTION

The following description includes the preferred best mode of one embodiment of the present invention. It will be clear from this description of the invention that the invention is not limited to these illustrated embodiments but that the invention also includes a variety of modifications and embodiments thereto. Therefore, the present description should be seen as illustrative and not limiting. While the invention is susceptible to various modifications and alternative constructions, it should be understood, that there is no intention to limit the invention to the specific form disclosed, but, on the contrary, the invention is to cover all modifications, alternative constructions, and equivalents falling within the spirit and scope of the invention as defined in the claims.

In any embodiment described herein, the open-ended terms "comprising," "comprises," and the like (which are synonymous with "including," "having" and "characterized by") may be replaced by the respective partially closed phrases "consisting essentially of," consists essentially of," and the like or the respective closed phrases "consisting of," "consists of, the like.

As used herein, the singular forms "a," "an," and "the" designate both the singular and the plural, unless expressly stated to designate the singular only.

As used herein, the term, " Andrographolide" refers to an isolate extracted from *Andrographis paniculata,* which has been known to possess various pharmacological actions such as anti-microbial, anti-viral, anti-oxidant, immunostimulant, anti-diabetic, anti-infective, hepato-renal protective, anti-angiogenic & anti-allergic.

As used herein, the term, "2 Hydroxypropyl beta cyclodextrin" refers to an additive/excipient and most widely used molecule in the formulation development that plays important role in optimizing local and systemic drug delivery by enhancing drug release and/or permeation, stabilizing drugs in the formulation and alike.

The present invention relates to a bioactive formulation and method for preparation by combining a natural plant extract i.e. Andrographolide and hydroxyl propyl beta cyclodextrin to provide a anti-viral and immunomodulating formulation that enhances the immunity and play a crucial role in the inhibition of SAR CoV-2 virus as well as management of the clinical symptoms of COVID-19 disease.

The bioactive formulation comprises of: i) ethanolic concentrates of Andrographolide extract in the range of 90% by wt- 99% by wt, and ii) aqueous 2 hydroxypropyl beta cyclodextrin (HPBCD) in the range of 2% by wt- 10% by wt. The Andrographolide (AND) is extracted from *Andrographis paniculata.*

The amount of ethanolic concentrates of Andrographolide extract used herein is preferably 95% by wt and amount of aqueous 2 hydroxypropyl beta cyclodextrin (HPBCD) used is preferably 5%.

A method for preparation of the bioactive formulation involves the following steps: i) adding the Andrographolide extract and ethanol in a first beaker followed by mixing to obtain ethanolic concentrates of Andrographolide (solvent A), ii) adding the hydroxypropyl Beta cyclodextrin (HPBCD) and ethanol in a second beaker followed by mixing to obtain an ethanolic concentrate of 2 hydroxypropyl beta cyclodextrin (Solvent B), iii) mixing solvent A and solvent B for 1-3 hours at room temperature to obtain a complex concentrate solution i.e. Andrographolide and 2 hydroxypropyl beta cyclodextrin, iv) recovering ethanol solvent from the concentrate solution through distillation to obtain a thick paste of the complex concentrate, v) drying the thick paste in a vacuum tray drier at a temperature in the range of 55°C -65°C and pressure in the range of -650to 700mmHg to obtain a dried complex concentrate, and vi) converting the dried complex concentrate into an amorphous powder to obtain a final bioactive formulation.

The obtained bioactive formulation possess anti-viral and immunomodulating properties as well as plays crucial role in the inhibition of SARS CoV-2 VIRUS growth as well as in the prophylaxis and management of clinical symptoms of COVID-19 disease as Andrographolide (AG) extract known to possess wide spectrum of pharmacological properties such as, anti-microbial, anti-viral, anti-oxidant, immunostimulant, anti-diabetic, anti-infective, hepato-renal protective, anti-angiogenic & anti-allergic.

Andrographolide (AG) has been known to be used in the clinical management of upper respiratory tract infections (such as common cold, uncomplicated sinusitis, bronchitis & pharyngotonsillitis), fever, laryngitis and several infectious diseases like jaundice, malaria, dysentery etc.

### EXAMPLE

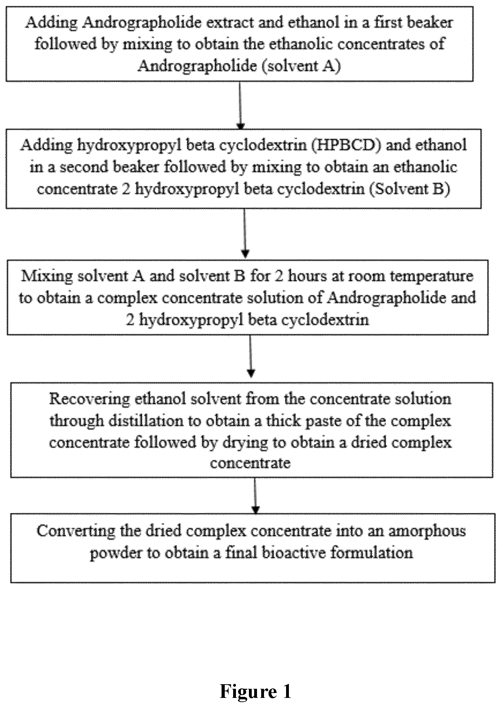

Referring to figure 1, a flow chart representation of the method for preparation of the bioactive formulation is represented which comprises of the following steps: i) Andrographolide extract and ethanol are added in a first beaker followed by mixing to obtain the ethanolic concentrates of Andrographolide (solvent A), ii) hydroxypropyl beta cyclodextrin (HPBCD) and ethanol are added in a second beaker followed by mixing to obtain an ethanolic concentrate of 2 hydroxypropyl beta cyclodextrin (Solvent B), iii) solvent A and solvent B are mixed for 2 hours at room temperature to obtain a complex concentrate solution i.e. Andrographolide and 2 hydroxypropyl beta cyclodextrin, iv) ethanol solvent is recovered from the concentrate solution through distillation to a thick paste of the complex concentrate, v) thick paste is dried in a vacuum tray drier at a temperature in the range of 60°C under vacuum in the range of - 650 mmHg to obtain a dried complex concentrate, and vi) the dried complex concentrate is converted into an amorphous powder to obtain a final bioactive formulation.

The Andrographolide is extracted & purified from *Andrographis paniculata* through various processing steps which are as follows:
i) *Andrographis paniculata* herb is weighed followed by grinding to obtain a fine herb (i.e. fineness upto10#), ii) then the herb is extracted in a four extraction process, wherein in a first extraction process, 1000kg of the fine herb is added in 6KL (kiloliter) rotary extractor followed by 5 times addition of pure ethanol to obtain a solvent mixture, iii) the solvent mixture is subjected to extraction process in the extractor at 60°C for 3 hrs under circulation to obtain a first ethanolic herbal extract, iv) the herbal extract is transferred in a clean storage tank through on-line-filter, v) 3 more extraction second, third and fourth extraction processes are repeated same as first process and mixed with the first herbal extract to obtain a combined solution of herbal extract, vi) solvent recovery from combined first, second, third and fourth ethanolic herbal extract is done by distillation at 60°C under vacuum up to 2000lit to obtain a concentrate, vii) 60 kg of activated charcoal (pH 5-6) is added in the concentrate followed by washing of Charcoal with RO water to remove soluble impurity.

Further viii) the charcoal treated concentrate is refluxed for 1 hour at 70°C, ix) charcoal treated concentrate is filtered through filter press/Nutsche filter on a hyflo super cell bed to remove chlorophylls and plant lipids, x)charcoal is refluxed 2 more times for 30 minutes each with 500 liter of pure ethanol followed by filtering to obtain a charcoal treated clear filtered extract (2000+500+500lit), xi) the filtered extract is concentrated under pressure upto 250 liter (25% of *Andrographis paniculata*) followed by storing at 10°C for48 hrs for crystallization to obtain a crystallized mass, xii)crystallized mass is filtered through Nutsche filter to obtain filtered crystallized mass, xiii) the filtered mass is lixiviated by refluxing with double volume of pure ethanol for 30 minutes at 70°C to obtain lixiviate mass, xiv) the lixiviate mass is unloaded in storage tank for 48 hrs at 10°C for recrystallization to obtain lixiviate crystallized mass, xv)lixiviate crystallized mass is filtered through nutsche filter followed by flushing with 5litre of pure cold ethanol to obtain a white crystallized mass, xvi) the filtered mass is dried in vacuum tray drier at 60°C under vacuum in the range of -650 mmHg to obtain a dried mass, xvii) the dried mass is grinded and sieved through 60# to obtain a sieved material, xviii) the sieved material is mixed in a blender and directly packing into high density polyethylene (HDPE) drums with double polybags to obtain a Andrographolide 95% powder.

To improve the bioavailability & solubility of AG, to assess the safety of formulation through oral and nasal delivery in animal and to prove the final bioactive formulation possess antiviral activity against SARS-CoV-2. This final bioactive formulation may boost the immune system of the person as well as may inhibit the SARS-CoV-2 virus growth and support in the clinical management of symptoms of COVID-19 disease.

The bioactive formulation was further characterized, determination of its oral bioavailability, in vitro antiviral activity against SARS-CoV2 virus and Acute & Sub-acute Oral & Inhalation toxicity study, which includes i) phase solubility, ii) attenuated total reflectance (ATR), iii) Nuclear Magnetic Resonance (1H NMR), iv) rotating frame overhause effect spectroscopy (ROESY), v) in silico docking analysis, vi) differential scanning calorimetry (DSC), vii) Powdered X-ray diffraction (PXRD), viii) Scanning electron microscopy (SEM), ix) Assay of synthesized solid complex, x) Solubility analysis, xi) dissolution testing, xii) Pharmacokinetic analysis, xiii) in vitro antiviral activity against SARS-CoV2, xiv) Acute oral toxicity study, xv) Sub-acute oral toxicity study xvi) Acute inhalation toxicity study and xvi) Sub-acute inhalation toxicity study.

### Phase solubility

Phase solubility assay was performed to compute the stoichiometry of the complex in the solution state. 10 mg of Andrographolide was suspended separately in 10 ml of aqueous phase containing 2-8Mm of aqueous 2 hydroxypropyl beta cyclodextrin (2-HP-β-CD) powder. All samples were then constantly stirred in an orbital shaker at 100 rpm for 5 consecutive days at 37±2°C. After equilibration, the samples were passed separately through a 0.22-µm membrane filter and analyzed for Andrographolide content by high performance liquid chromatography (HPLC) equipped with C8 column (4.6 x 2.5 cm, 5 µm). Stock solution of Andrographolide was prepared by dissolving the compound in methanol. Further, diluent (50% methanol and 50% water) was employed to get the concentration range of 10 to 70-µg/ml of Andrographolide. The mobile phase was formulated with 60% methanol and 40% water. The detection was carried out at absorption maxima of 225 nm. All measurements were carried out in triplicate (n=3).

From the analysis, it was observed that the solubility of Andrographolide increased linearly as a function of concentration of 2-HP-β-CD which clearly represented formation of a 1:1 complex of Andrographolide with 2-HP-β-CD.

### Characterization of solid complex

### Attenuated Total Reflectance (ATR)

The spectrum of Andrographolide, 2-HP-β-CD, bioactive formulation (1:1mM) complex was recorded between 4000 and 400 cm⁻¹ with a resolution of 4 cm⁻¹. Spectra were recorded to analyze the alteration of stretching frequencies due to hydrophobic association during the molecular encapsulation of Andrographolide in 2-HP-β-CD cavity.

It was observed that the ATR spectrum of Andrographolide demonstrated a characteristic peak at 1,723 cm⁻¹ (v, C=O), indicating the presence of a lactone ring. In addition, bioactive formulation peaks were observed at 1,633 cm⁻¹ and 1,074 cm⁻¹ that suggested presence of -C=C and -C-O bond, respectively. Further, peaks observed for Andrographolide were 2,925 cm⁻¹ and 2,849 owing to the presence of various OCH₃/CH₃ groups. The spectrum of 2-HP-β-CD revealed the vibration of free -OH groups at 3,343 cm⁻¹ whereas 2,927 cm⁻¹ and 1,022 cm⁻¹ indicated the presence of -CH₃ stretching and C-O-C bending, respectively. The physical mixture of Andrographolide with 2-HP-β-CD indicated the presence of identical peaks of individual components. Finally, spectrum of bioactive formulation suggested the synthesis due to the presence of intact lactone (1727 cm⁻¹) and -C=C- bond (1673 cm⁻¹) of drug in addition to 2925 cm⁻¹ (-CH₂/CH₃) and 3338 cm⁻¹ (-OH group) of 2-HP-β-CD.

### Nuclear Magnetic Resonance (¹H NMR) and Rotating Frame Overhause Effect spectroscopy (ROESY)

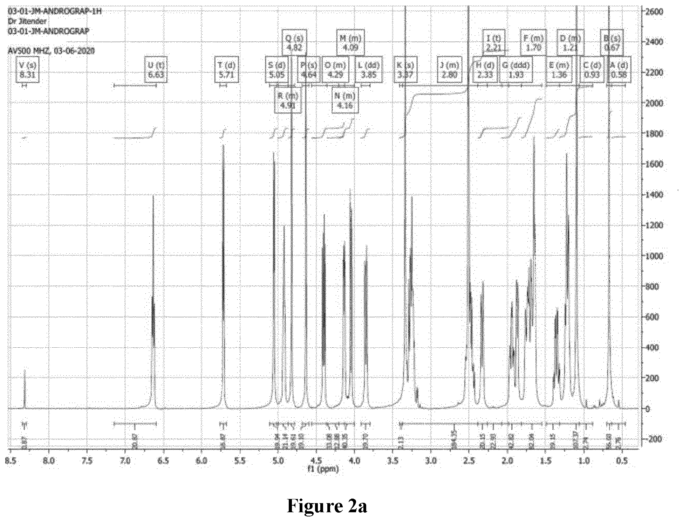

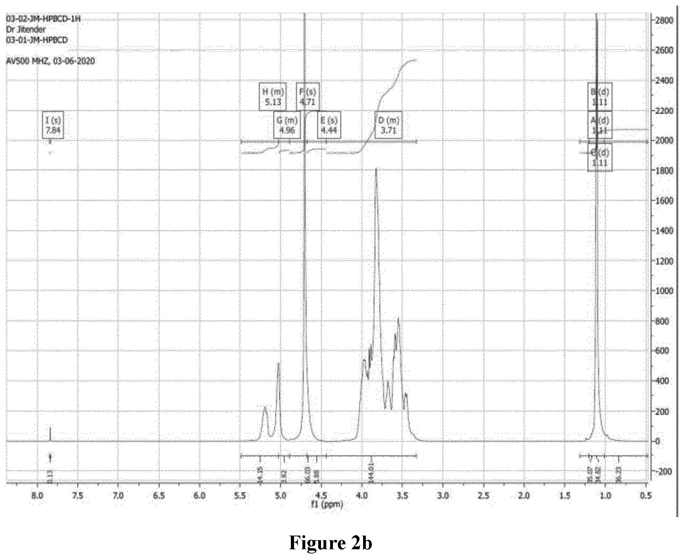

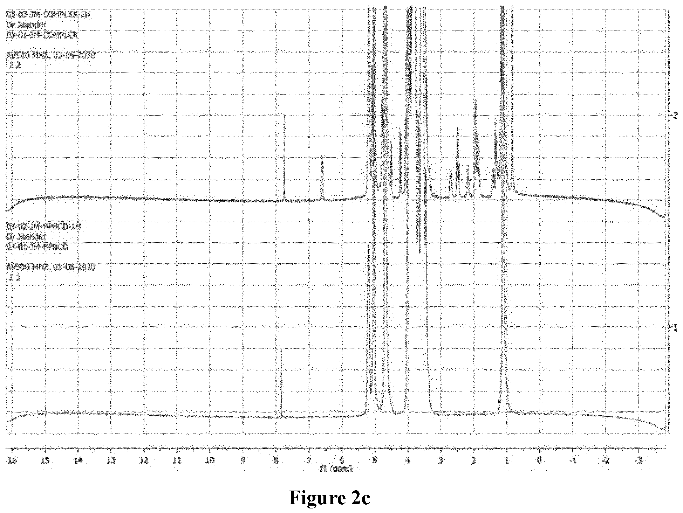

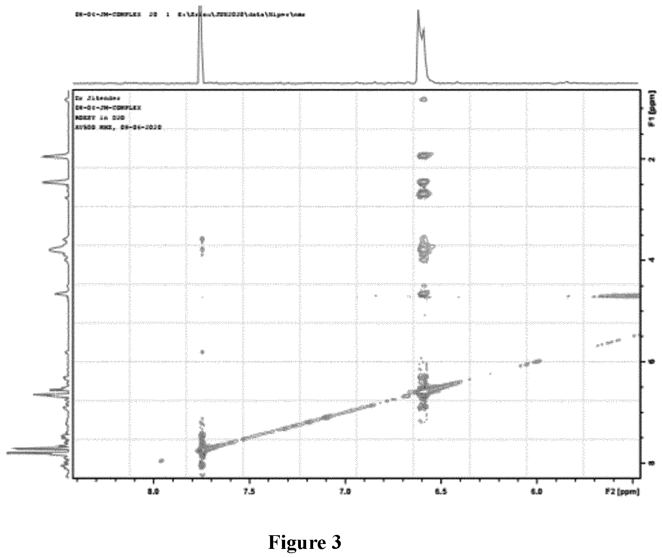

Referring to figure 2a, 2b, 2c and 3, a graphical representation of ¹H NMR spectra of Andrographolide, 2-HP-β-CD, bioactive formulation and 2d NMR (ROSEY) of bioactive formulation is represented. ¹H NMR spectra were recorded on BRUKER DPX 500 MHz spectrometer to analyze the molecular interactions within complex. The solution of AND was prepared in deuterated dimethyl sulfoxide (DMSO-d6) while 2-HP-β-CD and bioactive formulation were prepared separately in deuterated water (D₂O) and then transferred to NMR tubes. The 2D ROESY spectrum of bioactive formulation was recorded with a sweep width of 8.5 ppm in both dimensions. The total ROESY mixing time was set to 150 ms. The spectrum was acquired with 32 scans, 2048 data points in t₂ and 512 free induced decays (FIDs) in t₁.

It was observed that, according to the chemical shift variations, ¹H NMR communicates data on free and bound phases of a guest compound. The resultant chemical shift, Δδ, is represented as variation between bound and free guest molecule chemical shifts. Such resultant shifts were measured by applying the formula Δδ=δ_{complex}-δ_{free}. The positive and negative signs based on this equation indicated downfield and upfield shifts, respectively. Since (H₃ and H₅) protons (3.71 ppm) located in the cavity of 2-HP-β-CD were found to shift upfield (3.62 ppm) due to interaction with guest molecule, Andrographolide revealing the formation of complex through the inclusion mode. Also, the shift in the signals for the protons H₁, H₂, H₄, and H₆ existing on the exterior of 2-HP-β-CD indicated the host molecule's conformational change in the presence of guest compound. ¹H NMR spectrum of Andrographolide indicated the presence of triplet at 6.63 ppm which was up-fielded in the bioactive formulation at 6.60 ppm (doublet) confirmed the insertion of Andrographolide in cavity of 2-HP-β-CD through lactone ring. Andrographolide interactions with 2-HP-β-CD were also evaluated by ¹H-¹H 2D ROESY. The relative intensities of cross-peaks depend on the distance between the corresponding protons. ROESY correlates the triplet at 6.63 ppm of Andrographolide with H-3 protons (3.71 ppm) of 2-HP-β-CD.

### In silico docking analysis

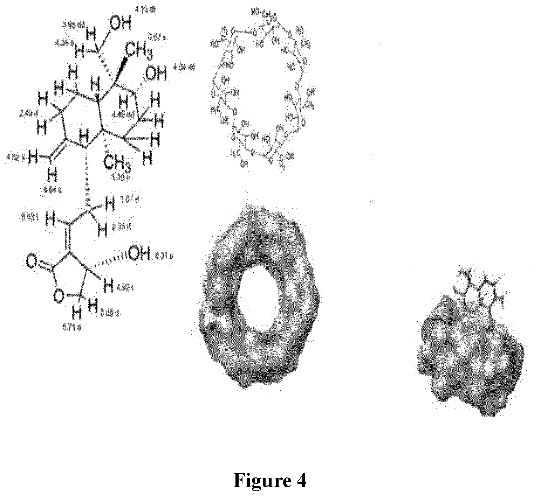

Referring to figure 4, in silico docking analysis of the bioactive formulation is represented. Molecular docking of Andrographolide with 2-HP-β-CD was performed using GLIDE docking module from the Schrödinger Suite. Extra precision (XP) docking calculations were carried out and the parameters of scaling factor and partial charge cutoff were set at the default values of 0.80 and 0.15, respectively.

The docking studies revealed that the 2-hydroxy-ethylidene furanone part of Andrographolide was posing within the cavity of 2-HP-β-CD. The docked pose of the Andrographolide exhibited a combination of electrostatic and hydrophobic non-bonded interactions that have stabilized the bioactive formulation. On the other hand, hexahydro naphthalenyl segment of Andrographolide remained outside the 2-HP-β-CD cavity. The binding affinity of Andrographolide with 2-HP-β-CD was measured to be -5.026 kcal/mol. It was observed that Andrographolide was best docked to 2-HP-β-CD with Glide energy of -29.807 kcal/mol, Potential energy of -3535.08 kcal/mol, Van der Waals energy of -6797.27 kcal/mol and electrostatic energy of -4060.78 kcal/mol. Further, contribution from Van der Waals energy was higher as compared to electrostatic energy for bioactive formulation stabilization as indicated in **Table 1.**

**Table 1: Parameters for the AND 2-HP-β-CD complex calculated using force field OPLS3**

| **S.No.** | **Parameters** | **Score kcal/mol** | **Glide Parameters** | **Score kcal/mol** |
|---|---|---|---|---|
| 1. | Potential energy | -3535.08 | Glide energy | -29.807 |
| 2. | Stretch energy | 301.838 | Glide Ligand efficiency, sa | -0.201 |
| 3. | Bend energy | 1275.981 | Glide Ligand efficiency, In | -1.191 |
| 4. | Dihedral energy | 1156.933 | Glide g Score | -5.026 |
| 5. | Electrostatic energy | -4060.78 | Glide docking Score | -5.026 |
| 6. | Van der Waals energy | -6797.27 | Glide e-model | -37.919 |

### Differential scanning calorimetry (DSC)

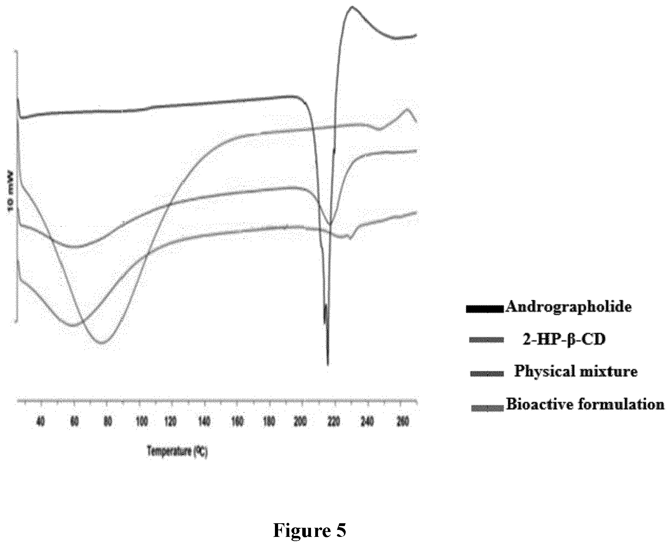

Referring to figure 5, graphical representation of differential scanning electroscopy is represented. DSC was measured through DSC-7, Mettler Toledo in order to measure endothermic peaks and comparison was made with individual components. The thermogram of Andrographolide, 2-HP-β-CD, physical mixture of AND with 2-HP-β-CD (1:1), and bioactive formulation was captured at a heating rate of 10°C/min by purging a nitrogen gas at a temperature in the range of 30 to 260°C.

It was observed that, the endothermic peak of Andrographolide was viewed at 214.81°C close to the melting range (225 to 226 °C). Thermograms of all the cyclodextrins (CDs i.e. α-, β and γ-CDs) display a broad range of endothermic peaks ranging from 40 to 150°C (77.52°C for 2-HP-β-CD) due to the dehydration process. The thermogram of the physical mixture of Andrographolide with 2-HP-β-CD indicated the presence of identical peaks of individual components. However, thermograms of bioactive formulation complex exhibited a complete disappearance of the endothermic peak characteristic of Andrographolide and a new peak of bioactive formulation complex was appeared at 60.40°C.

### Dissolution testing

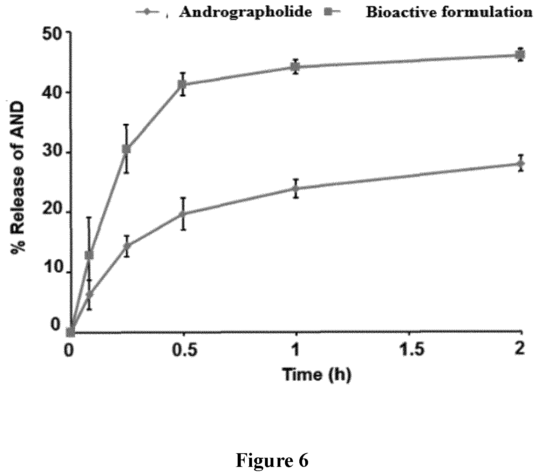

Referring to figure 6, a graphical representation of dissolution rate is represented. Dissolution testing were conducted as per the standard protocol using USP dissolution rate test apparatus with HPLC method (Sajeeb BK, 2015), wherein 100 mg of Andrographolide (AND) or 100 mg of AND equivalent in bioactive formulation was suspended in 900 ml of simulated intestinal fluid (SIF, pH 7.2) and paddle was rotated at 50 rpm. The temperature of SIF was maintained at 37±2°C. At specified time intervals, 2 ml of SIF was taken and replaced with identical volume. Subsequently, all the samples were filtered through 0.22 µm membrane filter and assayed for drug content at 225 nm.

It was observed that, Andrographolide demonstrated 27.89±1.32% release from gelatin capsule after 2 h, whereas bioactive formulation showed significantly (P<0.05) higher release (45.91±0.99%) at the same time interval.

### Powder X-ray diffraction (PXRD) and Scanning electron microscopy (SEM)

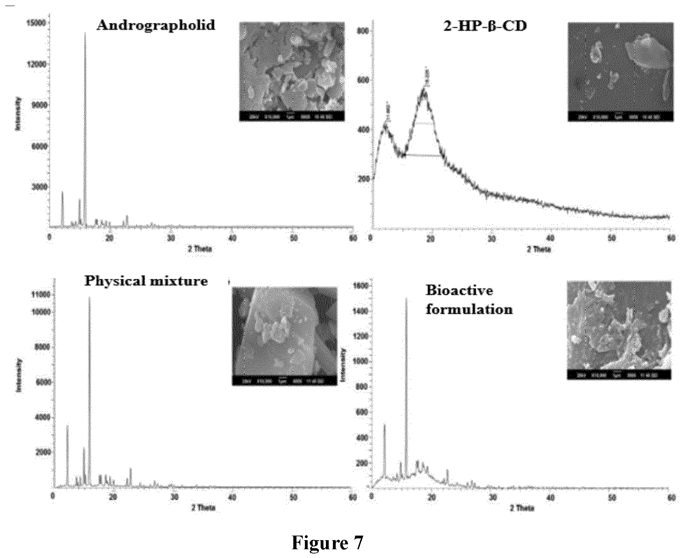

Referring to figure 7 a graphical representation of PXRD and SEM analysis of Andrographolide, 2-HP-β-CD, physical mixture of Andrographolide with 2-HP-β-CD (1:1 mM), and bioactive formulation is represented. PXRD analysis was conducted by using Rotaflex, RV 200 (Rigaku Corp., Japan) powder XRD using Ni-filtered, Cu Ka-radiation, a voltage of 40 kV, and a current of 40 mA. Each sample was scanned at 1°/min over 0-60° diffraction angle (2θ) range.

Diffractogram of Andrographolide displayed sharp peaks indicating its crystalline structure and that of 2-HP-β-CD demonstrated undefined broad and diffused peaks which signified reduction of crystalline structure, however, few sharp peaks were also observed indicating the presence of Andrographolide. Finally, PXRD of the bioactive formulation displayed broad diffused peaks of diminished intensities with limited sharp peaks. This confirmed the significant transformation of Andrographolide crystalline architecture into amorphous geometry in the bioactive formulation.

For SEM analysis, a film of Andrographolide 2-HP-β-CD, physical mixture of Andrographolide with 2-HP-β-CD (1:1 mM), and bioactive formulation was made on an aluminium stub. The stubs were then coated with gold with thickness of 200 to 500 Å under an argon atmosphere using a gold sputter module in a high vacuum evaporator. The coated samples were scanned and photographs were captured.

Presence of crystalline particles of irregular size of Andrographolide is observed. 2-HP-β-CD also appeared as particles with no definite structure. On the other hand, physical mixture showed the crystalline structure of both Andrographolide and 2-HP-β-CD instead of bioactive formulation which displayed small size particles tending to aggregation, suggested the existence of an amorphous product with the presence of a single component in the complex, and thus indicated the complete complexation.

### Bioactive formulation assay

The assay of solid complex was conducted through HPLC method (Sajeeb BK, 2015), wherein 50 mg of bioactive formulation was dissolved in 100 ml of methanol. Subsequently 1 ml of the sample was diluted to 10 ml with diluent (50%:50%, water: methanol) and filtered through 0.22 µm membrane filter. The filtrate was assayed for drug content at 225 nm. The experiment was carried out in triplicate (n=3). Presence of 17.03±0.60% of Andrographolide was observed in the bioactive formulation.

### Solubility Analysis

Solubility of the bioactive formulation was measured by saturated solution method (Madan *et al.* 2014), wherein 30 mg of bioactive formulation was suspended in 10 ml of aqueous phase and kept at 37±1°C at 100 rpm in an incubator orbital shaker for 24 hrs. After that, suspension was filtered and 0.1 ml of the filtrate was diluted with water up to 1 ml. The contents in the aliquot were measured by HPLC method (Sajeeb BK, 2015). All measurements were carried out in triplicate (n=3).

The solubility of the bioactive formulation observed was 17.15±1.52 µg/ml in aqueous phase, whereas pure Andrographolide exhibited significantly (Unpaired t test, *P*<0.05) lower solubility of 6.39±0.47 µg/ml. This represented a 2.68-fold increase in the aqueous solubility of AND upon complexation.

### Pharmacokinetic analysis

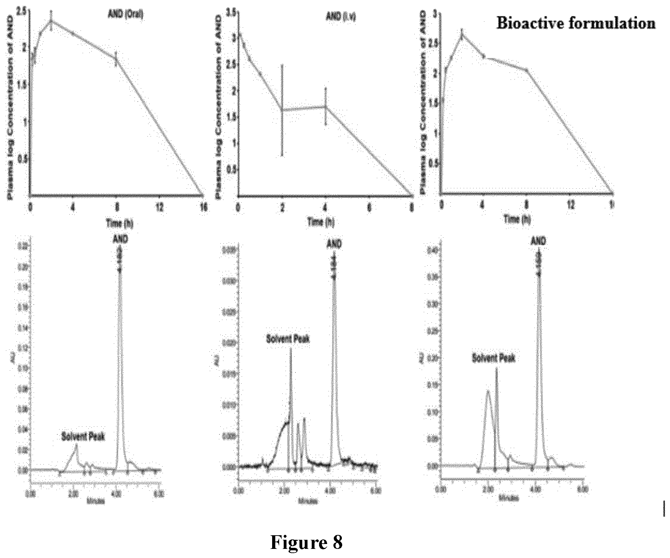

Referring to figure 8, a graphical representation of pharmacokinetic analysis of the bioactive formulation is represented. For pharmacokinetic analysis, male Sprague Dawley rats were used, wherein the animals were housed in controlled environment (23±1°C, 50±5% humidity) and allowed access to food and water *ad libitum.* The photoperiod of 12 h was maintained. All experiments were performed in accordance with the guidelines of Committee for the Purpose of Control and Supervision of Experiments on Animal (CPCSEA), Government of India. The study approved by IEC (Institutional Ethics Committee) committee of National Institute of Pharmaceutical Education and Research (NIPER), Hyderabad. 120 male rats were randomly divided into 30 groups of 4 animals each corresponding to the time points of blood collection. Andrographolide (AND) and AND equivalent to 100-mg/kg in bioactive formulation were administered orally by gavages. Rats were fasted (Approximately 4 h) before administration of drug. On the other hand, AND at the dose of 10-mg/kg was administered intravenously (i.v.) for comparative studies. Plasma concentration data was analyzed and computed for pharmacokinetic parameters. The following pharmacokinetic parameters were assessed: ke (h), t1/2 (h), Cmax (µg/ml), AUClast (h.µg/ml), AUCinf (h.µg/ml), MRT (h), CLTotal (L/h), Vd (L) and F%.

The log plasma concentration-time profile of Andrographolide (AND) and bioactive formulation was computed and presented in **Table 2.** Cₘₐₓ of AND depicted was 239.6±75.37 µg/ml at tₘₐₓ of 2 h upon oral administration of 100 mg/kg in male rats which was significantly (Unpaired t test, *P*<0.05) increased (461.105±103.00 µg/ml) in bioactive formulation indicating the improved absorption of drug **(Table 2).** However, the biological half-life of AND (5.92±0.21 h) was slightly shifted to higher value in bioactive formulation (6.38±0.055 h). In addition, bioactive formulation substantiated the improved absorption in terms of AUCₗₐₛₜ of 1842.115±125.51 h.µg/ml in comparison (Unpaired t test, P<0.05) of 1223.6±143.09 h.µg/ml of AND. The bioavailability (F%) was calculated and observed to be 15.87±3.84% and 23.84±5.46% for AND and bioactive formulation, respectively.

**Table 2: In vitro antiviral activity against SARS-Co V2**

| **Parameters** | **AND (Oral, 100 mg/kg)** | **AND (i.v., 10 mg/kg)** | **Bioactive complex Complex(Oral, 100 mg/kg)** |
|---|---|---|---|
| Ke (h) | 0.117±0.004 | 0.807±0.02 | 0.1085±0.001 |
| t ½ (h) | 5.92±0.21 | 0.859±0.031 | 6.38±0.055 |
| Cmax (µg/ml) | 239.6±75.37 | NA | 461.105±103.00 |
| AUC last (h. µg/ml) | 1223.6±143.09 | 801.14±181.07 | 1842.115±125.51 |
| AUC inf (h. µg/ml) | 1845.58±305.77 | 891.08±179.75 | 2937.37±108.10 |
| MRT (h) | 1.49±0.0.085 | 1.11±0.04 | 1.59±0.05 |
| CLTotal (L/h) | 0.055±0.008 | 0.011±0.002 | 0.032±0.002 |
| Vd (L) | 0.468±0.060 | 0.013±0.003 | 0.299±0.019 |
| F (%) | 15.87±3.84 | NA | 23.84±5.46% |

### In vitro antiviral activity against SARS-CoV2

The AND-2-HP-β-CD complex did not show any cytotoxcity to Vero E6 cells at 10-µg/ml or 100-µg/ml, measured by standard cell proliferation assay. AND-2-HP-β-CD complex at the concentration of 9 mg/ml demonstrated 85.97% (E) and 85.89% (N) inhibition of Vero E6 cells infected with SARS-CoV2 at 24 significantly (One Way ANOVA test, P<0.05) higher than 72.26% (E) and 77.61% (N) presented by standard drug, remdesivir. Correspondingly, AND-2-HP-β-CD complex at the concentration of 0.9 mg/ml demonstrated 86.38% (E) and 85.95% (N) inhibition of Vero E6 cells infected with SARS-CoV2 at 24 h significantly (One Way ANOVA test, P<0.05) higher than 72.26% (E) and 77.61% (N) presented by standard drug, remdesivir. On the other hand, there was no significant (One way ANOVA test, P>0.05) difference in antiviral efficacy of AND-2-HP-β-CD complex at 9 mg/ml or 0.9 mg/ml in comparison to remdesivir at 48 h. The IC50 of remdesivir was estimated to be 0.09 µg/ml for E gene and 0.06 µg/ml for N gene significantly (One way ANOVA test, P<0.05) lower than 0.1 µg/ml (E gene) and 0.29 µg/ml (N gene) exhibited by AND-2-HP-β-CD complex.

### Acute Oral Toxicity Study

Acute oral toxicity study was performed on female Sprague Dawley rats. The animals were housed in the controlled environment (19.1 - 22.7°C Room Temperature, 39-65% Relative Humidity) and allowed access to food and water *ad libitum.* The photoperiod of 12 h was maintained. All experiments were performed in accordance with the OECD 423 guidelines (Organisation for Economic Co-operation and Development). The study approved by IEC (Institutional Ethics Committee) committee of National Institute of Pharmaceutical Education and Research (NIPER), Hyderabad. In brief, 3 healthy female rats were selected and were administered orally with 200 mg/kg of bioactive formulation dosage by gavages. Rats were fasted for 16 h prior to dosing. Dosing was made using a stainless-steel feeding needle. Animals were observed individually after dosing at least once during the first 30 min for first 4 hours and every 4 hours thereafter for next 24 hours. After 24 hours, rats were observed each day for 14 days. The observation was made with regard to clinical signs, gross behavioural changes, gross pathology and mortality along with assessment of food intake and body weight.

Bioactive formulation at the dose equivalent to 2000 mg/kg in rat was found to be safe. Hence LD50 (Lethal dose) was found to be 2000 mg/kg as per the OECD 423 guidelines. All the major organs of rats were found normal in size and appearance. Any abnormalities of appearance or behaviour or other signs of reaction to treatment or ill health were recorded during study and has been found normal behaviour with no clinical signs.

### Sub-acute Oral Toxicity Study

Sub-acute oral toxicity study was performed on male and female Sprague Dawley rats. The animals were housed in controlled environment (20±3°C Room Temperature, 35-68% Relative Humidity) and allowed access to food and water *ad libitum.* The photoperiod of 12 h was maintained. All experiments were performed in accordance with the OECD 407 guidelines (Organisation for Economic Co-operation and Development). The study approved by IEC (Institutional Ethics Committee) committee of National Institute of Pharmaceutical Education and Research (NIPER), Hyderabad. 72 total healthy male and female Sprague Dawley rats were divided into 6 groups. All the animals were observed for mortality and morbidity for a period of twenty-eight days for main study and 14 additional days for evaluating reversal effect of bioactive formulation. Animals of different treatment groups and doses were administered bioactive formulation orally at dose volume of 1ml/100 gm body weight. Animals were assessed for their behavioral changes after each exposure. Moreover, after the exposure period on 28 day, hematological and biochemical parameters were investigated. Animals were evaluated for anxiety, motor activity and other behavioral changes.

From the sub-acute oral toxicity studies, it was observed that bioactive formulation at the dose equivalent to 666 mg/kg in rat was found to be safe. Hence LD50 (Lethal dose) was found to be 666mg/kg as per OECD 407 guidelines. All the major organs of rats were found normal in size and appearance. Any abnormalities of appearance or behaviour or other signs of reaction to treatment or ill health were recorded during study and has been found normal behaviour with no clinical signs. Acute Inhalation Toxicity Study

Acute inhalation toxicity study was performed on female Sprague Dawley rats. The animals were housed in the controlled environment (19.1 - 22.7°C Room Temperature, 39-65% Relative Humidity) and allowed access to food and water ad libitum. The photoperiod of 12 h was maintained. All experiments were performed in accordance with the OECD 403 guidelines (Organisation for Economic Co-operation and Development). The study approved by IEC (Institutional Ethics Committee) committee of National Institute of Pharmaceutical Education and Research (NIPER), Hyderabad. In brief, 30 healthy female & male rats were selected and were administered through nasal with 5 mg/L/4 h (Andrographolide with 2 Hydroxypropyl beta cyclodextrin solution prepared in citrate buffer with pH 6.5) of bioactive formulation dosage by nebulizer. Animals were fasted for 4 h prior to dosing. Dosing was made using a standard nebulizer. Animals were observed individually after dosing at least once during the first 30 min for first 4 h and every 4 h, thereafter for next 24 hours. After 24 hours, animals were observed each day for 14 days. The observation was made with regard to clinical signs, gross behavioral changes and mortality along with assessment of food intake and body weight.

Bioactive formulation at the dose equivalent to 5 mg/L/4 h in rat was found to be safe. Hence MTD (Maximum Tolerable Concentration) was found to be >5 mg/L/4 h. All the major organs of rats were found normal in size and appearance and no abnormalities has been found in appearance or behaviour or other signs of reaction to treatment or ill health were recorded during study and has been found normal behaviour with no clinical signs.

### Sub-acute Inhalation Toxicity Study

Sub-acute inhalation toxicity study was performed on male and female Sprague Dawley rats. The animals were housed in controlled environment (20±3°C Room Temperature, 35-68% Relative Humidity) and allowed access to food and water ad libitum. The photoperiod of 12 h was maintained. All experiments were performed in accordance with the OECD 413 guidelines (Organisation for Economic Co-operation and Development). The study was approved by IEC (Institutional Ethics Committee) committee of National Institute of Pharmaceutical Education and Research (NIPER), Hyderabad. Total 60 healthy male and female Sprague Dawley rats were divided into 10 groups. All the animals were observed for mortality and morbidity for a period of twenty-eight days. Animals of different treatment groups and doses were administered bioactive formulation through nebulization at low dose (1/10 of MTC), Mid dose (1/5 of MTC) and High dose (1/3 of MTC). Satellite groups are required only for solid aerosols as per OECD 412 (TG). Animals were assessed for their behavioral changes after each exposure. Moreover, after the exposure period on 28 day, hematological and biochemical parameters were investigated. Animals were evaluated for anxiety, motor activity and other behavioral changes. Any abnormalities of appearance or behaviour or other signs of reaction to treatment or ill health were recorded during study.

Although the field of the invention has been described herein with limited reference to specific embodiments, this description is not meant to be construed in a limiting sense. Various modifications of the disclosed embodiments, as well as alternate embodiments of the invention, will become apparent to persons skilled in the art upon reference to the description of the invention.

## Claims

1. A bioactive formulation, comprising:
**i)** ethanolic concentrates of Andrographolide extract in the range of 90% by wt- 99% by wt; and
**ii)** aqueous 2 hydroxypropyl beta cyclodextrin (HPBCD) in the range of 2% by wt- 10% by wt.

2. The formulation as claimed in claim 1, wherein said Andrographolide is extracted from *Andrographis paniculata.*

3. A method for preparation of said bioactive formulation as claimed in Claim 1, comprising the steps of;
i. adding said Andrographolide extract and ethanol in a first beaker followed by mixing to obtain said ethanolic concentrates of Andrographolide (solvent A);
ii. adding said hydroxypropyl beta cyclodextrin (HPBCD) and ethanol in a second beaker followed by mixing to obtain said ethanolic concentrate 2 hydroxypropyl beta cyclodextrin (Solvent B);
iii. mixing said solvent A and solvent B for 1-3hours at room temperature to obtain a complex concentrate solution i.e. Andrographolide and 2 hydroxypropyl beta cyclodextrin;
iv. recovering ethanol solvent from said concentrate solution through distillation to obtain a thick paste of said complex concentrate;
v. drying said thick paste at a temperature in the range of 55°C-65°C and pressure in the range of -650 to 700mmHg to obtain a dried complex concentrate; and
vi. converting said dried complex concentrate into an amorphous powder to obtain a final bioactive formulation.

4. The method as claimed in claim 3, wherein said drying is performed in a vacuum tray drier.

5. The method as claimed in claim 3, wherein said amorphous powder is converted into said dried complex concentrate by grinding and sieving said complex concentrate.
